(19) Europäisches Patentamt
European Patent Office
Office européen des brevets

(11) Veröffentlichungsnummer: **0 052 305**
**A1**

(12) # EUROPÄISCHE PATENTANMELDUNG

(21) Anmeldenummer: **81109477.0**

(22) Anmeldetag: **02.11.81**

(51) Int. Cl.³: **C 07 D 251/34**
C 08 G 18/79, C 08 G 18/77

(30) Priorität: **13.11.80 DE 3042820**

(43) Veröffentlichungstag der Anmeldung:
**26.05.82 Patentblatt 82/21**

(84) Benannte Vertragsstaaten:
**BE DE FR GB IT**

(71) Anmelder: **BAYER AG**
**Zentralbereich Patente, Marken und Lizenzen**
**D-5090 Leverkusen 1, Bayerwerk(DE)**

(72) Erfinder: **Schwindt, Jürgen, Dr.**
**Kleist-Platz 4**
**D-5090 Leverkusen(DE)**

(72) Erfinder: **Riberi, Bernd, Dr.**
**Jakob-Böhme-Strasse 2**
**D-5000 Koeln 80(DE)**

(72) Erfinder: **Grögler, Gerhard, Dr.**
**von-Diergardt-Strasse 46**
**D-5090 Leverkusen(DE)**

(72) Erfinder: **Bock, Manfred, Dr.**
**Haydnstrasse 18**
**D-5090 Leverkusen(DE)**

(54) **Neue, schwefelhaltige Isocyanato-Isocyanurate, ein Verfahren zu ihrer Herstellung und ihrer Verwendung als Aufbaukomponente bei der Herstellung von Polyurethankunststoffen.**

(57) Die Erfindung betrifft Isocyanato-Isocyanurate der Formel

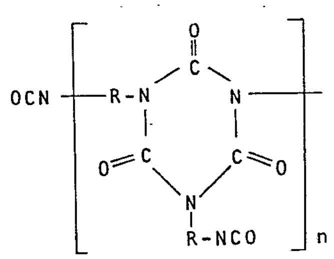

in welcher
R für einen Rest der Formel

$-R'-S-R''-$

steht, wobei
R' für einen gegebenenfalls inerten Substituenten aufweisenden Phenylenrest und
R'' für einen gesättigten aliphatischen Kohlenwasserstoffrest mit 2 bis 12 Kohlenstoffatome, wobei zwischen der freien Bindung und dem Schwefelatom mindestens 2 Kohlenstoffatome angeordnet sind, oder einen gegebenenfalls inerte Substituenten aufweisenden para-Phenylen-Rest stehen, und
n eine ganze Zahl oder (im statistischen Mittel) gebrochene Zahl von 1 bis 5 steht,

ein Verfahren zur Herstellung dieser Verbindungen durch teilweise Trimerisierung der Isocyanatgruppen der ihnen zugrundeliegenden Diisocyanate, sowie ihre Verwendung als Isocyanatkomponente bei der Herstellung von Polyurethankunststoffen.

EP 0 052 305 A1

BAYER AKTIENGESELLSCHAFT 5090 Leverkusen, Bayerwerk

Zentralbereich
Patente, Marken und Lizenzen Wr-by-c

Neue, schwefelhaltige Isocyanato-Isocyanurate, ein Verfahren zu ihrer Herstellung und ihrer Verwendung als Aufbaukomponente bei der Herstellung von Polyurethankunststoffen

Die vorliegende Erfindung betrifft neue Schwefel- und Isocyanuratgruppen aufweisende Polyisocyanate, ein Verfahren zu ihrer Herstellung durch die an sich bekannte teilweise Trimerisierung der Isocyanatgruppen der ihnen zugrundeliegenden Diisocyanate, sowie ihre Verwendung als Aufbaukomponente zur Herstellung von Polyurethankunststoffen nach dem Isocyanat-Polyadditionsverfahren.

Verfahren zur Trimerisierung organischer Polyisocyanate sind in großer Zahl bekannt (J.H. Saunders, R.C. Frisch, Polyurethanes Chemistry and Technology, S. 94 ff. 1962). Als Katalysatoren zur Trimerisierung der organischen Polyisocyanate werden neben tertiären Aminen, Metallsalzen organischer Säuren, vor allem Phosphine der aliphatischen und gemischt

aliphatisch-aromatischen Reihe sowie Mannichbasen verwendet, wie z.B. in DE-PS 1 201 992, GB-PS 949 253 oder den DE-OS'en 2 551 634, 2 641 380 oder 2 722 400 beschrieben wird.

Die auf diese Weise hergestellten Isocyanato-Isocyanurate, insbesondere jene auf Basis der in der Polyurethanchemie üblichen großtechnischen Diisocyanate wie z.B. 2,4-Diisocyanatotoluol, 4,4'-Diisocyanatodiphenylmethan, Hexamethylendiisocyanat oder 1-Isocyanato-3,3,5-trimethyl-5-isocyanatomethyl-cyclohexan stellen wertvolle modifizierte Polyisocyanate dar, die für die unterschiedlichsten Anwendungsgebiete, wie z.B. Lacke, Klebstoffe, Herstellung von Polyurethanschaumstoffen Eingang in die Polyurethanchemie fanden. Für viele Anwendungsgebiete ist es dabei nicht zuletzt auch aus physiologischen Gründen wesentlich, daß die Isocyanato-Isocyanurate in weitgehend monomerenfreier Form zur Verfügung stehen. Dies wird in der Praxis im allgemeinen dadurch erreicht, daß nach dem Abbruch der Trimerisierungsreaktion bei einem gewünschten Trimerisierungsgrad das überschüssige Ausgangsisocyanat insbesondere destillativ entfernt wird.

Wie jetzt überraschend gefunden wurde eignen sich bestimmte, nachstehend näher beschriebene, Schwefel enthaltende Diisocyanate besonders gut zur Herstellung von Isocyanuratgruppen aufweisenden Polyisocyanaten mit einem besonders niedrigen Gehalt an Ausgangsisocyanat, so daß oft eine destillative Entfernung des Ausgangsisocyanats entfallen kann. Die bei der Trimerisierung der genannten Schwefel enthaltenden Diisocyanate anfallenden, nachstehend näher beschriebenen erfindungsgemäßen Isocyanato-Isocyanurate weisen außerdem eine Reihe anwendungstechnisch besonders wertvoller Eigenschaften auf. So stellen beispielsweise die erfindungsgemäßen Isocyanate-Isocyanurate transparente Polyisocyanate dar, die sich auch zu transparenten Lösungen in gängigen Lacklösungsmitteln lösen lassen. Überraschend war außerdem die Feststellung, daß sich mit den erfindungsgemäßen Isocyanato-Isocyanuraten hergestellte Polyurethankunststoffe erheblich flammwidriger erwiesen als vergleichbare Polyurethankunststoffe auf Basis von Isocyanuraten aus Diisocyanatotoluol oder Diisocyanatodiphenylmethan.

Gegenstand der vorliegenden Erfindung sind Isocyanato-Isocyanurate der Formel

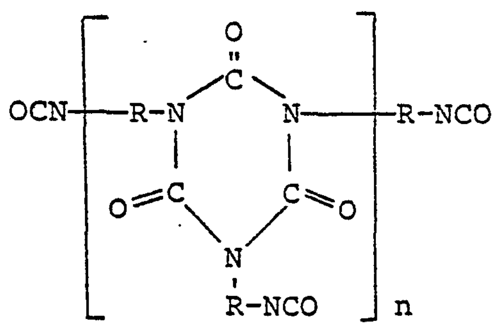

in welcher

R für einen Rest der Formel

-R'-S-R"-

steht, wobei

R' für einen gegebenenfalls inerten Substituenten aufweisenden Phenylenrest und

R" für einen gesättigten aliphatischen Kohlenwasserstoffrest mit 2 bis 12 Kohlenstoffatomen, wobei zwischen der freien Bindung und dem Schwefelatom mindestens 2 Kohlenstoffatome angeordnet sind, oder einen gegebenenfalls inerte Substituenten aufwendenden para-Phenylen-Rest stehen, und

n eine ganze oder (im statistischen Mittel) gebrochene Zahl von 1 bis 5 steht.

Gegenstand der vorliegenden Erfindung ist auch ein Verfahren zur Herstellung dieser Isocyanato-Isocyanurate durch an sich bekannte teilweise Trimerisierung der Isocyanatgruppen der ihnen zugrundeliegenden Diisocyanate in Gegenwart von an sich bekannten, die Trimerisierung von Isocyanatgruppen beschleunigenden Katalysatoren und den Abbruch der Trimerisierungsreaktion bei dem jeweils gewünschten Trimerisierungsgrad durch thermische Zersetzung des Katalysators und/oder durch Zugabe eines Katalysatorengifts, welches dadurch gekennzeichnet ist, daß man als zu trimerisierende Diisocyanate solche der Formel

OCN-R'-S-R"-NCO

einsetzt, wobei

R' und R" die obengenannte Bedeutung haben.

Gegenstand der vorliegenden Erfindung ist schließlich auch die Verwendung der erfindungsgemäßen Isocyanato-Isocyanurate als Isocyanatkomponente bei der Herstellung von Polyurethankunststoffen nach dem Isocyanat-Polyadditionsverfahren.

Ausgangsmaterialien für das erfindungsgemäße Verfahren sind Diisocyanate der Formel

OCN-R'-S-R"-NCO

wobei

R' und R" die bereits genannte Bedeutung haben.

Als Substituenten für R' kommen beispielsweise Brom, Chlor, $-SO_2R'''$, $-OR'''$, $-SR'''$, $-SO_2N(R''')_2$ oder $-R'''$, wobei R"' für einen linearen oder verzweigten Alkylrest mit 1 bis 12, insbesondere 1 bis 4 Kohlenstoffatomen steht, in Betracht. Falls R" für einen p-Phenylenrest steht, kann dieser die gleichen Substituenten aufweisen.

Vorzugsweise werden beim erfindungsgemäßen Verfahren solche Diisocyanate der genannten allgemeinen Formel eingesetzt, für welche

R' für einen gegebenenfalls inerten Substituenten der genannten Art aufweisenden ortho-Phenylenrest und

R" für einen gegebenenfalls ebensolche Substituenten aufweisenden para-Phenylenrest oder einen Polymethylenrest mit 2 bis 6 Kohlenstoffatomen stehen, wobei in diesem Falle davon ausgegangen werden kann, daß wegen der sterischen Hinderung durch das Schwefelatom der ortho-ständigen Isocyanatgruppe diese allenfalls in untergeordnetem Umfang an der Trimerisierungsreaktion teilnimmt.

Besonders bevorzugte Ausgangsdiisocyanate sind die entsprechenden unsubstituierten Diisocyanate.

Typische Beispiele geeigneter Diisocyanate sind 2,4'-, 3,4'- oder 4,4'-Diisocyanatodiphenylsulfid, 2-(2-Isocyanatoethylthio)-phenylisocyanat, 2-(6-Isocyanatohexylthio)-phenylisocyanat, 2-(12-Isocyanatododecylthio)-phenylisocyanat, oder beliebige, an den Phenylenresten inerte Substituenten der genannten Art aufweisende Derivate der genannten Diisocyanate. Es ist auch möglich, beim erfindungsgemäßen Verfahren beliebige Gemische der beispielhaft genannten Diisocyanate einzusetzen, beispielsweise Gemische aus 2,4'- und 3,4'-Diisocyanatodiphenylsulfid in Gewichtsverhältnis von 4:1 bis 1:4, insbesondere 2:1 bis 1:2; Gemische aus 2,4'- , 3,4'- und 4,4'-Diisocyanatodiphenylsulfid im Gewichtsverhältnis 4:2:1 bis 1:4:1, vorzugsweise 2:1:1 bis 1:2:1. Gemische aus 2,4'- oder 3,4'- mit 4,4'-Diisocyanatodiphenylsulfid im Gewichtsverhältnis 4:1 bis 1:4, vorzugsweise 2:1 bis 1:1; Gemische aus 2-(2-Isocyanatoethylthio)-phenylisocyanat und 2-(6-Isocyanatohexylthio)-phenylisocyanat im Gewichtsverhältnis 4:1 bis 1:4, vorzugsweise 2:1 bis 1:2 oder Gemische der beispielhaft genannten Diisocyanate mit ausschließlich aromatisch gebundenen Isocyanatgruppen mit Diisocyanaten mit einer aromatisch und einer aliphatisch gebundenen Isocyanatgruppe im Gewichtsverhältnis 4:1 bis 1:4, vorzugsweise 2:1 bis 1:2.

Für das erfindungsgemäße Verfahren geeignete aromatische Diisocyanate, deren aromatische Kerne durch Schwefel verbunden sind und deren NCO-Gruppen in p-p'-Stellung zum Schwefelatom stehen sind bekannt (JA 74-048196; JA 71-38985). Diese und auch die sonstigen erfindungsgemäß geeigneten Diisocyanate können im übrigen nach dem bzw. in Analogie zu dem Verfahren der europäischen Patentanmeldung 80 101 845.8 bzw. der US-Patentanmeldung 136 624 vom 2.4.1980 hergestellt werden. Die Herstellung der Diisocyanate erfolgt in an sich bekannter Weise durch Phosgenierung der ihnen zugrundeliegenden Diamine. Die hierbei zum Einsatz gelangenden, Thioethergruppen aufweisenden Diamine mit einer aliphatisch und einer aromatisch gebundenen Aminogruppe sind beispielsweise in Analogie zum Verfahren der DE-OS 27 34 575 durch Umsetzung der entsprechenden Natrium-amino-thiophenolate der Formel

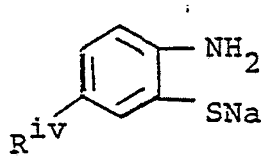

($R^{iv}$ = gegebenenfalls vorliegender indifferenter Substituent)

mit entsprechenden Chloraminen der Formel

$$Cl-R-NH_2$$

zugänglich, wobei die genannten Natriumthiophenolate auf einfache Weise durch alkalische Verseifung der entsprechenden Benzothiazole erhalten werden können.

Die ausschließlich aromatisch gebundene Aminogruppen und Thioethergruppen aufweisenden Diamine können beispielsweise durch Umsetzung der bereits genannten, o-aminosubstituierten Natrium-thiophenolate mit den entsprechenden, gegebenenfalls in o-Stellung zur Nitrogruppe Substituenten der oben beispielhaft genannten Art aufweisenden, p-Nitro-chlorbenzolen der Formel

$$Cl-R''-NO_2$$

zu den entsprechenden eine Amino- und eine Nitrogruppe aufweisenden Zwischenstufen umgesetzt werden, worauf sich eine Hydrierung der Nitrogruppe zur Aminogruppe, beispielsweise mittels Zink/Chlorwasserstoff oder unter Verwendung von Ranney-Nickel als Katalysator, anschließt. Die Herstellung der genannten Zwischenstufe wird in Prinzip beispielsweise in J. Chem. Soc. London 1930, 180 ff beschrieben.

Eine weitere Methode zur Herstellung von 2 aromatisch gebundene Aminogruppen aufweisenden Thioethern besteht beispielsweise in der Umsetzung der zuletzt genannten p-Nitrochlorbenzole mit Natriumsulfid zu den entsprechenden p-Amino-thiophenolaten der Formel

$$H_2N-R''-SNa$$

und deren anschließender Kondensation mit o-Chlornitrobenzolen der Formel

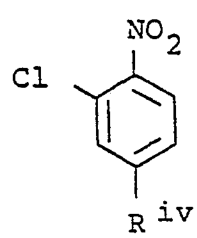

worauf sich auch hier wieder eine Hydrierung der noch vorliegenden Nitrogruppe anschließt. Die Herstellung der eine Aminogruppe und eine Nitrogruppe aufweisenden Zwischenstufe nach diesem Prinzip ist beispielsweise in J. Chem. Soc. London 1930, 180 beschrieben.

Schließlich sind die Thioethergruppen aufweisenden Diamine mit 2 aromatisch gebundenen Aminogruppen auch durch Umsetzung der entsprechenden o-Nitro-thiophenolate mit den entsprechenden p-Nitrochlorbenzolen bzw. durch Umsetzung der entsprechenden p-Nitrothiophenolate mit den entsprechenden o-Nitro-chlorbenzolen zu der 2 Nitrogruppen aufweisenden Zwischenstufe und deren anschließende Hydrierung zugänglich. Die Herstellung der bei dieser Methode vorliegenden Zwischenstufe ist beispielsweise in Journal of the American Chemical Society 45, 1399 ff beschrieben.

Die Trimerisierungsreaktion erfolgt in an sich bekannter Weise, beispielsweise wie in den Deutschen Patentschriften 951 168, 1 013 869, 1 112 285, 1 022 789, 1 203 792 sowie in den Britischen Patentschriften 809 809, 821 158, 837 120, 856 372, 927 173, 920 080, 952 931, 944 309, 954 095, 962 689, 966 338, 941 379, 949 253, in den Amerikanischen Patentschriften 3 154 522, 2 801 244, in der Französischen Patentschrift 1 510 342 oder in der Belgischen Patentschrift 718 994 beschrieben.

Die Trimerisierungsreaktion wird im allgemeinen bei 20° bis 70°C unter Verwendung der bekannten Trimerisierungskatalysatoren des Standes der Technik durchgeführt.

Dabei kann im allgemeinen die Trimerisierungsreaktion bei einem gewünschten Trimerisierungsgrad durch Zugabe von Katalysatorgiften abgebrochen werden. Im Falle der Verwendung von thermisch labilen Trimerisierungskatalysatoren kann der Abbruch der Trimerisierungsreaktion jedoch auch durch kurzzeitiges Erhitzen des Reaktionsgemischs auf eine Temperatur oberhalb der Zersetzungstemperatur des Katalysators erfolgen.

Im Falle der Verwendung von Katalysatorengiften werden die in den obengenannten Literaturstellen erwähnten Kombinationen Katalysator/Katalysatorengift verwendet. Vorzugsweise werden jedoch als Katalysatoren die in der DE-OS 2 551 634 genannten Mannichbasen als Katalysatoren sowie die in der gleichen Literaturstelle genannten Katalysatorengifte verwendet.

Das erfindungsgemäße Verfahren kann in Substanz oder in Lösung durchgeführt werden. Vorzugsweise erfolgt die Umsetzung in Gegenwart geeigneter inerter Lösungsmittel. Als Lösungsmittel kommen beliebige organische Lösungsmittel bzw. -gemische in Frage, die keine gegenüber Isocyanatgruppen reaktionsfähige Gruppen aufweisen und sowohl für die Ausgangsisocyanate als

auch für die Verfahrensprodukte Löser sind, und deren Siedepunkt im weiten Bereich von ca. 50°C/1013 mbar bis 250°C/13 mbar liegt. Je nach Anwendungsbereich der erfindungsgemäßen Verfahrensprodukte können niedrig- bis mittelsiedende Lösungsmittel oder hochsiedende angewandt werden. Bevorzugt können eingesetzt werden Ester, z.B. Ethylacetat, Butylacetat, Ethylglykolacetat oder Phthalsäureester, wie z.B. Dibutylphthalat, Butylbenzylphthalat, oder Phosphorsäureester, z.B. Trikresylphosphat, oder auch Alkylsulfonsäureester des Phenols und Kresols; ferner Ketone wie z.B. Aceton, Methylethylketon, Methylisobutylketon, Cyclohexanon und Methoxyhexanon sowie einige Chlorkohlenwasserstoffe wie z.B. Chloroform und Chlorbenzol.

Mit Verschnittmitteln wie z.B. Toluol, Xylol und höheren Aromaten besteht nur eine begrenzte Löslichkeit. Höhere Zusätze solcher Lösungsmittel können zu Trübungen und Ausfällungen in den Reaktionsprodukten führen.

Das bei der Herstellung der erfindungsgemäßen Isocyanate-Isocyanurate eingesetzte Lösungsmittel bzw. die eingesetzte Lösungsmittelmenge braucht nicht mit dem Lösungsmittel bzw. der Lösungsmittelmenge identisch zu sein, die in den erfindungsgemäßen Verfahrensprodukten bei ihrer erfindungsgemäßen Verwendung vorliegen. So kann man nach Beendigung des erfindungsgemäßen Verfahrens das eingesetzte

Lösungsmittel bzw. Lösungsmittelgemisch selbstverständlich ganz oder teilweise destillativ entfernen und ganz oder teilweise durch ein anderes Lösungsmittel ersetzen.

Die erfindungsgemäßen Isocyanate-Isocyanurate stellen wertvolle Ausgangsmaterialien zur Herstellung von Polyurethankunststoffen nach dem Isocyanat-Polyadditionsverfahren dar. Sie können entweder in Substanz oder als Lösung in geeigneten Lösungsmitteln, wie z.B. Ethylacetat, Butylacetat, Methylethylketon, Ethylglykolacetat oder Methylenchlorid verarbeitet werden und eignen sich zur Herstellung von Lacküberzügen, Polyurethanschaumstoffen oder massiven Formteilen auf Polyurethanbasis. Sie können gegebenenfalls im Abmischung mit den üblichen Polyisocyanaten der Polyurethanchemie verarbeitet werden. Insbesondere bei der Herstellung von Polyurethanschaumstoffen können die erfindungsgemäßen Isocyanato-Isocyanurate im Gemisch mit den ihnen zugrundeliegenden Diisocyanaten oder im Gemisch mit den üblichen Polyisocyanaten des Standes der Technik eingesetzt werden. Im allgemeinen kommen die erfindungsgemäßen Isocyanate-Isocyanurate jedoch in praktisch monomerenfreier Form, d.h. mit einem Gehalt an Ausgangsdiisocyanat von maximal 2 Gew.-% zum Einsatz.

Reaktionspartner der erfindungsgemäßen Isocyanate-Isocyanurate bei ihrer erfindungsgemäßen Verwendung sind insbesondere die in der Polyurethanchemie an

sich bekannten Verbindungen mit mindestens 2 aliphatisch gebundenen Hydroxylgruppen des Molekulargewichtsbereichs 62 bis 10 000, vorzugsweise 1 000 bis 6 000. Es handelt sich um die an sich bekannten einfachen mehrwertigen Alkohole wie z.B. Ethylenglykol, Propylenglykol, Tetramethylen-diol, Hexamethylendiol, Glycerin, Trimethylolpropan oder um Hydroxylgruppen aufweisende Polyesterpolyole, d.h. die Umsetzungsprodukte von mehrwertigen, vorzugsweise zweiwertigen und gegebenenfalls zusätzlich dreiwertigen Alkoholen mit mehrwertigen, vorzugsweise zweiwertigen, Carbonsäuren, sowie um die an sich bekannten Polyetherpolyole, die in an sich bekannter Weise durch Alkoxylierung geeigneter Startermoleküle mit Ethylenoxid und/oder Propylenoxid erhalten worden sind. Auch die Mitverwendung von an sich bekannten Polyaminen als Kettenverlängerungsmittel wie z.B. Toluylendiamin, Phenylendiamin, Diaminoethan, Hexamethylendiamin oder 4,4'-Diaminodiphenylmethan ist bei der erfindungsgemäßen Verwendung der erfindungsgemäßen Isocyanato-Isocyanurate möglich. Grundsätzlich können bei der erfindungsgemäßen Verwendung der erfindungsgemäßen Verbindungen alle beliebigen Verbindungen mit gegenüber Isocyanatgruppen reaktionsfähigen Wasserstoffatomen und alle in der Polyurethanchemie an sich bekannten Hilfs- und Zusatzmittel, wie diese beispielsweise in der europäischen Patentanmeldung 80 101 845.8 oder US-Patentanmeldung 136 624 vom 2.4.1980 beschrieben sind, mitverwendet werden.

Die in den nachstehenden Beispielen gemachten Prozentangaben beziehen sich auf Gewichtsprozente.

In den nachfolgenden Beispielen werden folgende Mannichbasen als Katalysatoren verwendet:

Mannichbase I

188 Gew.-Teile Phenol werden mit 720 Gew.-Teilen einer 25 %igen wäßrigen wäßrigen Dimethylamin-Lösung versetzt, anschließend werden im Verlauf von 30 Minuten noch 425 Gew.-Teile 40 %ige Formalinlösung zugesetzt. Man erhitzt 1 Stunde auf ca. 30°C und dann im Verlauf von weiteren 2 Stunden auf 80°C. Nach 2 Stunden bei 80°C trennt man durch Zugabe von Kochsalz die organische Phase von der wäßrigen Phase ab und engt die organische Phase bei 80° bis 90°C/20 mbar ein. Man erhält ca. 390 Gew.-Teile eines Kondensationsproduktes, das bei einem Stickstoffgehalt von 13,5 % eine Viskosität von ca. 500 mPas bei 25°C aufweist. Die Mannichbase stellt im wesentlichen ein Gemisch homologer Verbindungen dar. Das Gemisch enthält ca. 55 % der Mannichbase der Formel

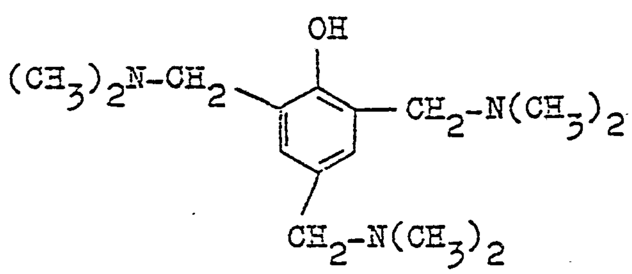

und ca. 20 % der Mannichbase der Formel

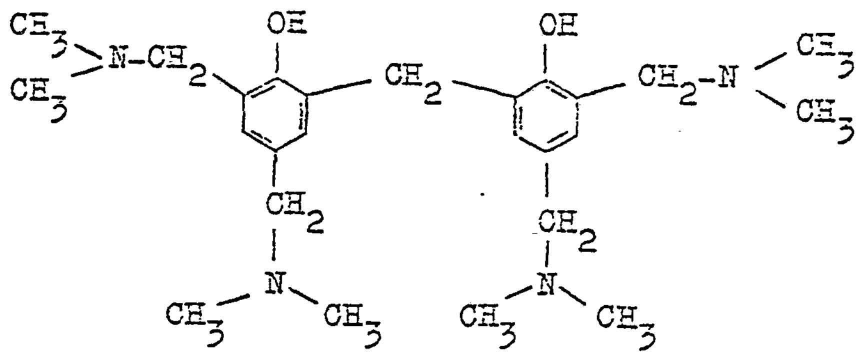

Mannichbase II

220 Gew.-Teile p-Isononylphenol und 45 Gew.-Teile Dimethylamin in Form einer 25 %igen wäßrigen Lösung werden bei etwa 25°C vorgelegt und dazu werden im Verlauf von 30 Minuten 30 Gew.-Teile Formaldehyd in Form einer 40 %igen wäßrigen Lösung hinzugefügt. Nach einer einstündigen Reaktionszeit bei 30°C wird die Temperatur im Verlauf von 2 Stunden auf 80°C erhöht und weitere 2 Stunden dabei belassen. Durch Zugabe von Kochsalz wird nunmehr die organische Phase von der wäßrigen Phase getrennt und die erstere bei 70°C/16 mbar eingeengt. Nach dem Einengen werden eventuell auftretende anorganische Bestandteile durch Filtration abgetrennt. Erhalten werden 264 Gew.-Teile Mannichbase mit einer Viskosität von 218 mPas bei 25°C.

Die Mannichbase ist im wesentlichen gekennzeichnet durch die Formel

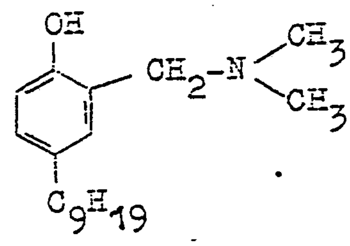

Nachfolgend wird die Herstellung von für das erfindungsgemäße Verfahren geeigneten Diisocyanaten beschrieben:

Diisocyanat I

Zu 1 Mol 2-Aminothiophenol-Na-Lösung (m-, p-) in 400 ml Wasser werden 200 ml Methanol hinzugefügt und bei 80°C eine warme Lösung von 1 Mol 4-Nitrochlorbenzol in 300 ml Methanol hinzugetropft. Man rührt 4 Stunden unter Rückfluß nach und kühlt ab. Es wird abgesaugt, mit Wasser gewaschen und getrocknet. 1 Mol der Nitroamino-Verbindung wird in Methanol gelöst und nach bekannten Verfahren mit Ra-Nickel zum Diamin hydriert.

In eine Lösung von 600 g Phosgen in 1,8 l Chlorbenzol werden bei -10 bis 0°C innerhalb von 35 Minuten 324 g (1,51 Mol) 2,4'-Diaminodiphenylsulfid, gelöst in 1,8 l Chlorbenzol eingetropft. Anschließend wird während eines Zeitraumes von 1,5 Stunden bis zum Rückfluß erhitzt

(130°C). Ab 80°C wird dabei weiteres Phosgen in die Lösung eingeleitet. Anschließend wird unter weiterem Einleiten von Phosgen 2 Stunden auf Rüclflußtemperatur erhitzt. Schließlich wird unter allmählichem Abkühlen der Reaktionslösung Phosgen durch 45-minütiges Einleiten von Stickstoff ausgespült, die Lösung eingeengt und das Produkt unter Vakuum destilliert. Es werden 361 g (1,35 Mol) 2,4'-Diisocyanatodiphenylsulfid (89,8 % der Theorie) erhalten.

| | | |
|---|---|---|
| NCO-Gehalt berechnet | = | 31,34 % |
| NCO-Gehalt gefunden | = | 31,3 % |
| Chlorgehalt | = | 0,05 % |

<u>Diisocyanat II</u>

Es handelt sich hierbei um 3,4'-Diisocyanato-diphenylsulfid, welches in Analogie zu Diisocyanat I unter Verwendung von 3-Aminothiophenol als Ausgangsmaterial hergestellt worden ist.

<u>Diisocyanat III</u>

Es handelt sich um 4,4'-Diisocyanato-diphenylsulfid, welches in Analogie zu Diisocyanat I unter Verwendung von 4-Aminothiophenol als Ausgangsmaterial hergestellt worden ist.

Diisocyanat IV

Es handelt sich um 2-(6-Isocyanatohexylthio)-phenylisocyanat, welches wie folgt hergestellt worden ist:

Zu 1 Mol 2-Aminothiophenol-Natrium, gelöst in 400 ml Wasser, werden 200 ml Methanol hinzugefügt und bei 70°C eine Lösung von 1 Mol 6-Chlor-hexylammoniumhydrochlorid in ca. 250 ml Wasser hinzugetropft. Man kocht 4 Stunden unter Rückfluß, kühlt ab und stellt mit Natronlauge stark alkalisch. Die organische Phase wird abgetrennt und im Hochvakuum destilliert.

Anschließend wird das so erhaltene Diamin wie oben beschrieben zu 2-(6-Isocyanatohexylthio)-phenylisocyanat mit einem Siedepunkt bei 0,5 mbar von 170°C phosgeniert.

Diisocyanate V und VI

Analog zu Diisocyanat IV werden 2-(2-Isocyanatoethylthio)-phenylisocyanat (Diisocyanat V) und 3-(2-Isocyanatoethylthio)-phenylisocyanat (Diisocyanat VI) hergestellt.

Beispiel 1

306 Gew.-Teile Diisocyanat I werden in 306 Gew.-Teilen Ethylglykolacetat gelöst und anschließend bei Raumtemperatur mit 0,54 Gew.-Teilen Mannichbase I vermischt. Die Trimerisierung setzt unter Erwärmung des Reaktionsgemisches sofort ein. Die Reaktionstemperatur wird auf 45°C eingestellt, das reagierende Gemisch bei dieser Temperatur 12 1/2 Stunden gerührt und eine Probe entnommen. Nach einer Gesamtreaktionszeit von 19 1/2 Stunden wird mit 1,2 ml o/p-Toluolsulfonsäuremethylester abgestoppt und 1 Stunde bei 60°C nachgerührt. Folgende Spezifikationswerte der Lösung werden erhalten.

| Reaktionszeit h | NCO gef.% | Monomerengehalt % bezogen auf Feststoff |
|---|---|---|
| 12 1/2 | 6,2 | 3 |
| 14 1/2 | 6,0 | 2 |
| 16 1/2 | 6,0 | 1,5 |
| 19 1/2 | 6,0 | 0,9 |

Viskosität $\eta$ 20°C = 5000 mPas

Vergleichsbeispiel 1

285,5 Gew.-Teile (1,142 Mol) 4,4'-Diisocyanato-diphenylmethan und 285,5 Gew.-Teile n-Butylacetat werden bei 4°C mit zweimal 0,5 ml einer Mannichbase aus i-Nonylphenol, Formaldehyd und Dimethylamin versetzt. Die Reaktion startet exotherm und wird anfänglich durch Kühlung, später durch Heizen bei einer Temperatur von 45°C gehalten. Nach einer Stunde tritt eine weiße Ausfällung auf. Der NCO-Gehalt des Gemisches beträgt dann 14,1 %. Nach weiteren 7,5 Stunden beträgt der NCO-Gehalt 6,9 %. Nach Abkühlung des Gemisches auf Raumtemperatur wird der Ansatz fest.

Vergleichsbeispiel 2

Anstelle von 4,4'-Diisocyanato-diphenylmethan, wie im Vergleichsbeispiel 1, wird ein Isomerengemisch aus ca. 60 % 2,4'-Diisocyanato-diphenylmethan und ca. 40 % 4,4'-Diisocyanato-diphenylmethan verwendet. Nach 8,5 Stunden gibt man 2 ml p-Toluolsulfonsäuremethylester zu und erhitzt 1 Stunde auf 60°C. Nach Abkühlen auf Raumtemperatur erhält man eine klare, schwach gelbgefärbte Lösung mit einer Viskosität von 1200 mPas (23°C). Der NCO-Gehalt beträgt 6,4 %. Der Monomerengehalt der Lösung beträgt 9,2 Gew.-%.

Beispiel 2

306 Gew.-Teile Diisocyanat I werden in 306 Gew.-Teilen wasserfreiem Butylacetat gelöst und bei Raumtemperatur mit 0,54 Gew.-Teilen Mannichbase I vermischt. Nach 12 Stunden Rühren bei 45°C ist der NCO-Gehalt der Lösung auf 5,95 % abgesunken. Anschließend wird die Lösung mit 1,2 ml o/p-Toluolsulfonsäuremethylester abgestoppt und 1 Stunde bei 60°C nachgerührt. Die Endspezifikationen der Lösung sind:

NCO-Gehalt : 5,9 %
Viskosität $\eta$ 20°C: 1150 mPas
freies 2,4'-Diisocyanato-diphenylsulfid : 1 % bezogen auf Feststoff

Beispiel 3

Beispiel 2 wird wiederholt jedoch unter Verwendung von 0,7 g der Mannichbase II. Nach 16 Stunden wird ein NCO-Gehalt der Lösung von 6,2 % NCO gefunden. Nach Abstoppen der Reaktion mit 1,4 ml o/p-Toluolsulfonsäuremethylester wird folgende Endspezifikation erhalten:

| | |
|---|---|
| NCO-Gehalt | : 6,15 % |
| Viskosität $\eta$ 20°C | : 1070 cp |
| freies 2,4'-Diisocyanato-diphenylsulfid | : 1,2 % bezogen auf Feststoff |

Beispiel 4

Beispiel 1 wird wiederholt, jedoch unter Verwendung von 306 Gew.-Teilen Diisocyanat II anstelle des Diisocyanats I. Nach 14 Stunden Reaktion bei 45°C wird abgestoppt und nachgerührt.

| | | |
|---|---|---|
| NCO-Gehalt | : | 6,25 % |
| $\eta_{20°C}$ | : | 3700 mPas |
| Monomeres | : | 1,7 % bezogen auf Feststoff |

Beispiel 5

306 Gew.-Teile eines Gemischs aus gleichen Teilen Diisocyanat I und Diisocyanat II werden in 306 Gew.-Teilen Ethylglykolacetat gelöst und bei Raumtemperatur mit 0,54 Gew.-Teilen Mannichbase I vermischt. Nach 14 Stunden Rühren bei 45°C ist der NCO-Gehalt der Lösung auf 6,38 % abgesunken. Anschließend wird die Lösung mit 1,2 ml o/p-Toluolsulfonsäuremethylester abgestoppt und 1 Stunde bei 60°C nachgerührt.

| | | |
|---|---|---|
| NCO-Gehalt | : | 6,12 % |
| $\eta_{20°C}$ | : | 4150 mPas |
| Monomerengehalt | : | 1,35 % bezogen auf Feststoff |

Beispiel 6

Beispiel 5 wird wiederholt, jedoch unter Verwendung von 306 Gew.-Teilen eines Gemischs aus gleichen

Teilen Diisocyanat I und Diisocyanat III. Nach 20 Stunden Reaktionszeit wird die folgende Spezifikation erreicht:

| | | |
|---|---|---|
| NCO-Gehalt | : | 6,4 % |
| Viskosität $\eta_{20°C}$ | : | 5500 mPas |
| Monomerengehalt | : | 2 % bezogen auf Feststoff |

<u>Beispiel 7</u>

Analoges Verfahren wie in Beispiel 5 mit 488 Gew.-Teilen eines Gemisches aus 268 Gew.-Teilen Diisocyanat I und 220 Gew.-Teilen Diisocyanat V, gelöst in 488 Gew.-Teilen Ethylglykolacetat, und 0,8 Gew.-Teilen Mannichbase I. Nach einer Reaktionszeit von 26 Stunden bei 45°C und nachfolgendem Abstoppen wird folgende Spezifikation erreicht:

| | | |
|---|---|---|
| NCO-Gehalt berechnet | : | 8,64 % |
| NCO-Gehalt | : | 7 % |
| Viskosität $\eta_{20°C}$ | : | 950 mPas |
| Monomerengehalt | : | 0,9 % bezogen auf Feststoff |

<u>Beispiel 8</u>

276 Gew.-Teile Diisocyanat IV werden in 276 Gew.-Teilen Ethylglykolacetat gelöst und bei Raumtemperatur mit 0,7 Gew.-Teilen Mannichbase I vermischt. Nach 26 Stunden Reaktionszeit bei 50°C ist der NCO-Gehalt der Lösung auf 6,8 % abgesunken. Anschließend wird die Lösung mit 1,3 ml o/p-Toluolsulfonsäuremethylester abgestoppt und 1 Stunde bei 70°C nachgerührt. Folgende Endspezifikation wird erreicht:

| | |
|---|---|
| NCO-Gehalt | : 6,65 % |
| Viskosität $\eta$ 20°C | : 750 mPas |
| Monomerengehalt | : 1,85 % bezogen auf Feststoff |

Beispiel 9

276 Gew.-Teile Diisocyanat IV und 220 Gew.-Teile Diisocyanat V werden in 496 Gew.-Teilen Ethylglykolacetat gelöst und bei Raumtemperatur mit 1,4 Gew.-Teilen Mannichbase I vermischt. Nach 28 Stunden bei 50°C ist der NCO-Gehalt auf 7,5 % abgesunken. Mit 2,6 ml o/p-Toluolsulfonsäuremethylester wird abgestoppt und 1 Stunde bei 70°C nachgerührt.

| | |
|---|---|
| NCO-Gehalt | : 7,45 % |
| Viskosität $\eta$ 20°C | : 800 mPas |
| Monomerengehalt | : 1,75 % bezogen auf Feststoff |

Beispiel 10

220 Gew.-Teile Diisocyanat V und 220 Gew.-Teile Diisocyanat VI werden in 440 Gew.-Teilen Ethylglykolacetat gelöst und mit 1,4 Gew.-Teilen Mannichbase II vermischt.

Nach 22 Stunden bei 50°C ist der NCO-Gehalt auf 8,1 % abgesunken. Mit 2,6 ml o/p-Toluolsulfonsäuremethylester wird abgestoppt und 1 Stunde bei 70°C nachgerührt.

| | |
|---|---|
| NCO-Gehalt | : 8,1 % |
| Viskosität η 20°C | : 810 mPas |
| Monomerengehalt | : 1,55 % bezogen auf Feststoff |

Beispiel 11

200 Gew.-Teile Diisocyanat V werden bei Raumtemperatur in 220 Gew.-Teilen Ethylglykolacetat gelöst und mit 1,7 Gew.-Teilen Triethylendiamin (DABCO) vermischt. Nach einer Reaktionszeit von 20 Stunden bei 70°C beträgt der NCO-Gehalt der lösung 8,7 %. Mit 2,6 ml o/p-Toluol-sulfonsäuremethylester wird abgestoppt und 1 Stunde bei 80°C nachgerührt.

| | |
|---|---|
| NCO-Gehalt | : 8,4 % |
| Viskosität 20°C | : 200 mPas |
| Monomerengehalt | : 2 % bezogen auf Feststoff |

Beispiele 12a und 12b

100 Gew.-Teile einer 75 %igen xylolischen Lösung eines Erdnußalkydharzes mit der OH-Zahl 146, bestehend aus dem Kondensationsprodukt von 29,8 Gew.-Teilen Erdnußfettsäure, 23,09 Gew.-Teilen Trimethylolpropan, 8,76 Gew.-Teilen Triethylenglykol,

34,92 Gew.-Teilen Phthalsäureanhydrid und 0,72 Gew.-Teilen Maleinsäureanhydrid werden bei Raumtemperatur mit

a) 71 Gew.-Teilen einer 60 %igen Lösung in Butylacetat eines Trimerisats auf Basis 2,4-Toluylendiisocyanat (Verhältnis NCO:OH = 1:1) und mit

b) 124 Gew.-Teilen einer 60 %igen Lösung in Butylacetat des Trimerisats nach Beispiel 3 (NCO:OH = 1:1) vermischt und zu Lackfilmen verarbeitet. Die Eigenschaften der Bindemittel wie der erhaltenen Filme sind in Tabelle 1 aufgeführt.

Tabelle 1

a) Anstieg der Viskosität (sec) gemessen im ISO-Becher nach 0 bis 48 h - DIN 53 224

| | 0h | 2h | 4h | 6h | 8h | 24h | 48h |
|---|---|---|---|---|---|---|---|
| 12a | 35 | 37 | 46 | 64 | 97 | - | - |
| 12b | 30 | 30 | 30 | 33 | 36 | 92 | - |

b) Grifftrocknung (min) und Pendelhärte nach König DIN 53 157

| Vers.Nr. | Filmdicke µm | Grifftrocknung min | Dämpfung in (sec) n. 1 d | 3 d | 7 d | 14 d |
|---|---|---|---|---|---|---|
| 12a | 90 | 25 | 178 | 193 | 197 | 195 |
| 12b | 90 | 35 | 163 | 185 | 186 | 188 |

c) Dehnbarkeit (mm) nach Erichsen, Filmdicke, 30 bis 40 µm - DIN 53 156

| Vers. Nr. | Dehnbarkeit mm |
|---|---|
| 12a | 0 |
| 12b | 2 |

| d) Vers.Nr. | Lösungsmittel | Anlösbarkeit nach 1d | 3d | 7d | 14d | Bewertung |
|---|---|---|---|---|---|---|
| 12a | Xylol | 0 | 0 | 0 | 0 | 0 = Film nicht angegriffen |
| 12b | | 0 | 0 | 0 | 0 | |
| 12a | Butylacetat | 3 | 1 | 0 | 0 | 5 = Film zerstört |
| 12b | | 2 | 1 | 0 | 0 | |
| 12a | Ethylacetat | 3 | 2 | 1 | 0 | |
| 12b | | 3 | 2 | 2 | 1 | |
| 12a | Aceton | 5 | 5 | 5 | 5 | |
| 12b | | 5 | 4 | 4 | 3 | |

Patentansprüche

1. Isocyanato-Isocyanurate der Formel

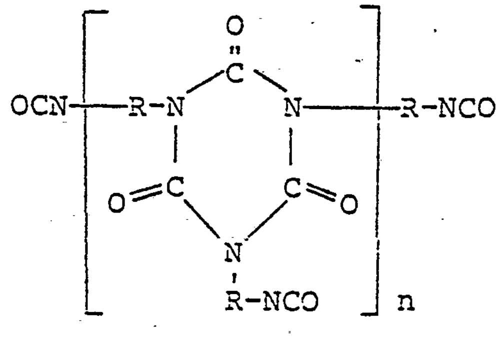

in welcher

R für einen Rest der Formel

-R'-S-R"-

steht, wobei

R' für einen gegebenenfalls inerten Substituenten aufweisenden Phenylenrest und

R" für einen gesättigten aliphatischen Kohlenwasserstoffrest mit 2 bis 12 Kohlenstoffatomen, wobei zwischen der freien Bindung und dem Schwefelatom mindestens 2 Kohlenstoffatome angeordnet sind, oder einen gegegebenenfalls inerte Substituenten aufweisenden para-Phenylen-Rest stehen, und

n eine ganze oder (im statistischen Mittel) gebrochene Zahl von 1 bis 5 steht.

2. Isocyanato-Isocyanurate der in Anspruch 1 genannten allgemeinen Formel, dadurch gekennzeichnet, daß

R' für einen ortho-Phenylen-Rest und

R" für einen para-Phenylen-Rest stehen und

n die in Anspruch 1 genannte Bedeutung hat.

3. Isocyanato-Isocyanurate der in Anspruch 1 genannten Formel, dadurch gekennzeichnet, daß

R' für einen ortho-Phenylen-Rest und

R" für einen Polymethylenrest mit 2 bis 6 Kohlenstoffatomen stehen und

n die in Anspruch 1 genannte Bedeutung hat.

4. Verfahren zur Herstellung von Isocyanato-Isocyanuraten gemäß Anspruch 1 bis 3 durch teilweise Trimerisierung der Isocyanatgruppen der ihnen zugrundeliegenden Diisocyanate in Gegenwart von an sich bekannten, die Trimerisierungsreaktion von Isocyanatgruppen beschleunigenden

Katalysatoren und Abbruch der Trimerisierungsreaktion bei dem jeweils gewünschten Trimerisierungsgrad durch thermische Zersetzung des Katalysators und/oder durch Zugabe eines Katalysatorengiftes, dadurch gekennzeichnet, daß man als Diisocyanate solche der Formel

OCN-R'-S-R"-NCO

einsetzt, wobei

R' und R" die in Anspruch 1 bis 3 genannte Bedeutung haben.

5. Verwendung der Isocyanato-Isocyanurate gemäß Anspruch 1 bis 3 als Isocyanatkomponente bei der Herstellung von Polyurethankunststoffen nach dem Isocyanat-Polyadditionsverfahren.

0052305

Europäisches Patentamt

# EUROPÄISCHER RECHERCHENBERICHT

Nummer der Anmeldung
EP 81 10 9477

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | DE - A - 1 222 067 (FARBENFABRIKEN BAYER A.G.)<br>* Anspruch *<br>-- | 1 |
| A | DE - A - 2 727 650 (ASAHI GLASS CO. LTD.)<br>* Ansprüche *<br>-- | |
| A | US - A - 3 409 618 (THE DOW CHEMICAL COMPANY)<br>* Seite 1 *<br>---- | |

KLASSIFIKATION DER ANMELDUNG (Int. Cl.3)

C 07 D 251/34
C 08 G 18/79
18/77

RECHERCHIERTE SACHGEBIETE (Int. Cl.3)

C 07 D 251/34

KATEGORIE DER GENANNTEN DOKUMENTE

X: von besonderer Bedeutung allein betrachtet
Y: von besonderer Bedeutung in Verbindung mit einer anderen Veröffentlichung derselben Kategorie
A: technologischer Hintergrund
O: nichtschriftliche Offenbarung
P: Zwischenliteratur
T: der Erfindung zugrunde liegende Theorien oder Grundsätze
E: älteres Patentdokument, das jedoch erst am oder nach dem Anmeldedatum veröffentlicht worden ist
D: in der Anmeldung angeführtes Dokument
L: aus andern Gründen angeführtes Dokument

&: Mitglied der gleichen Patentfamilie, übereinstimmendes Dokument

✓ Der vorliegende Recherchenbericht wurde für alle Patentansprüche erstellt.

| Recherchenort | Abschlußdatum der Recherche | Prüfer |
|---|---|---|
| Den Haag | 27-01-1982 | VAN BIJLEN |